# FASCICULE DE BREVET EUROPEEN

(11) **EP 0 667 897 B1**
(45) Date de publication et mention de la délivrance du brevet: **01.08.2001**
(21) Numéro de dépôt: 94926264.6
(22) Date de dépôt: 01.09.1994
(51) Int. Cl.: C12N 9/08, C07K 16/40, G01N 33/573

(54) **METHODE D'IMMUNODOSAGE SPECIFIQUE DE LA GLUTATHION PEROXYDASE PLASMATIQUE HUMAINE**
SPEZIFISCHES IMMUNOTESTSYSTEM FÜR MENSCHLICHE PLASMA GLUTATHIONPEROXIDASE
METHOD FOR SPECIFIC IMMUNOASSAY OF HUMAN PLASMATIC GLUTATHIONE PEROXIDASE

(30) Priorité: 03.09.1993 FR 9310504
(43) Date de publication de la demande: 23.08.1995
(73) Titulaire: OXIS Isle of Man, Limited, Douglas, Isle of Man IM1 (GB)
(72) Inventeur: CHAUDIERE, Jean, F-94100 Saint-Maur (FR); LEMAINQUE, Arnaud, F-77380 Combs-la-Ville (FR); MALETTE, Patricia, F-94100 Saint-Maur (FR)
(74) Mandataire: Portal, Gérard
(86) Numéro de dépôt international: FR9401031
(87) Numéro de publication internationale: WO9506719

(56) Documents cités:
- EP-A- 0 392 369
- JOURNAL OF BIOCHEMISTRY., vol.108, 1990, TOKYO JP pages 145 - 148 K. TAKAHASHI ET AL 'Primary structure of human plasma glutathion peroxidase deduced from cDNA sequences' cité dans la demande
- PROCEEDINGS OF THE NATIONAL ACADEMY OF SCIENCES OF USA., vol.81, Juillet 1984, WASHINGTON US pages 3998 - 4002 H.M. GEYSEN ET AL 'Use of peptide synthesis to probe viral antigens for epitopes to a resolution of a singel amino acid'
- PROCEEDINGS OF THE NATIONAL ACADEMY OF SCIENCES OF USA., vol.82, Janvier 1985, WASHINGTON US pages 178 - 182 H.M. GEYSEN ET AL 'Small peptides induce antibodies with a sequence and structural requirement for binding antigen comparable to antibodies raised against the native protein'

## Description

Méthode d'immunodosage spécifique de la glutathion peroxydase plasmatique humaine, kit pour sa mise en oeuvre, oligopeptides et anticorps spécifiques de la méthode.

La présente invention concerne une méthode de dosage de la glutathion peroxydase plasmatique (pl.GPx) humaine et une trousse prête à l'emploi pour la mise en oeuvre de ladite méthode ; elle est basée sur la sélection de séquences peptidiques de la pl.GPx et la synthèse in vitro desdites séquences ainsi que sur la production et l'utilisation d'anticorps dirigés contre lesdites séquences et qui reconnaissent spécifiquement la pl.GPx.

Trois types de glutathion peroxydase humaine ont été caractérisés: la glutathion peroxydase plasmatique (pl.GPx), la glutathion peroxydase intra-cellulaire (c.GPx) essentiellement active sur les substrats hydrophiles et la glutathion peroxydase active sur les hydroperoxydes de phospholipides ainsi que sur d'autres substrats lipidiques (PHGPx). Ces enzymes catalysent la réduction des hydroperoxydes (H2O2 ou ROOH) par le glutathion (GSH). Ces enzymes sont des sélénoprotéines qui comportent une sélénocystéine au sein de leur site actif. La c.GPx et la pl.GPx sont des homotétramères tandis que la PHGPx est exclusivement monomérique [Biochim. Biophys. Acta, 839, 62-70 (1985)].

Initialement purifiée à partir du plasma [J. Biol. Chem., 262, N°36, 17398-17403 (1987); Arch. Biochem. Biophys., 256, N°2, 677-686 (1987)] la pl.GPx représente 0,007% de la masse protéique plasmatique totale. La pl.GPx a également été isolée à partir du lait maternel humain [J. Nutr., 121, n°8, 1243-1249 (1991)].

De nature glycoprotéique, la pl.GPx se présente sous forme d'un homotétramère d'un poids moléculaire de 94 kDa [J. Biol. Chem., 262, N°36, 17398-17403 (1987)]. Chaque sous-unité, caractérisée par un poids moléculaire de 21,5 à 23 kDa [J. Biol. Chem., 262, N°36, 17398-17403 (1987); J. Biochem., 108, 145-148 (1990)] contient une sélénocystéine dans son site actif.

Récemment, une étude réalisée chez le rat a mis en évidence que la pl.GPx est majoritairement synthétisée par la cellule rénale (Yoshimura et al. J. Biochem 109; 1991; 918-923) tandis qu'une autre étude a montré qu'une lignée de cellules hépatiques tumorales (Hep G2) d'origine humaine était capable de synthétiser cette pl.GPx (AVISSAR et al. J. Biol. Chem. 264; 1989; 15850-15855).

Compte-tenu des faibles concentrations de glutathion plasmatique, le rôle principal de la pl.GPx reste incertain. L'activité spécifique de la pl.GPx est environ dix fois inférieure à celle de la c.GPx [Arch. Biochem. Biophys., 256, N°2, 677-686 (1987)].

La séquence primaire de la pl.GPx humaine a été déterminée à partir de l'ADNc et publiée par Takahashi et al.[J. Biochem., 108; 1990; 145-148]. Il n'a pas été possible de séquencer totalement la pl.GPx et d'identifier l'extrémité N-terminale en raison de la glycosylation qui porte sur cette extrémité. Le nombre exact de résidus par sous-unité demeure donc inconnu.

La pl.GPx diffère considérablement de la c.GPx sur le plan structural: absence de glycosylation et de ponts disulfure intramoléculaires pour la forme cellulaire [Arch. Biochem. Biophys., 256, 677-686 (1987); Blood, 73, 318-323 (1989)], faible homologie de séquence entre les deux enzymes (de l'ordre de 44% [Nucleic Acids Res., 15; 1987, 5484; Nucleic Acids Res., 15; 1987; 7178]).

La mesure du taux de pl.GPx dans le plasma est un bon indicateur d'un éventuel état de carence en sélénium de l'organisme. Dans le cadre d'expériences destinées à quantifier l'influence d'une carence puis d'un apport en sélénium chez l'homme, il a été montré que les taux moyens de glutathion peroxydase ne varient pas à la même vitesse selon qu'il s'agit de la forme plasmatique ou de la forme cellulaire. Ainsi, le taux de pl.GPx dans le plasma commence à croître dès les premiers jours de traitement pour atteindre une valeur normale au bout de deux à quatre semaines. Le temps nécessaire au rétablissement de la valeur du taux normal de c.GPx est plus long: trois à quatre mois [Amer. J. Clin. Nutr., 41, 1985; 735-747].

De plus, et suite aux études précédemment réalisées chez le rat, un certain nombre de pathologies rénales pourraient être décelées par la mesure de pl.GPx.

Aucune des méthodes connues de dosage d'activité enzymatique ne permet actuellement de doser spécifiquement la pl.GPx dans les fluides biologiques ou dans des extraits tissulaires.

La méthode de dosage d'activité glutathion peroxydase la plus couramment utilisée implique un couplage enzymatique faisant intervenir la régénération du glutathion réduit par la glutathion réductase présente en excès; cette réaction s'accompagne de l'oxydation d'un cofacteur réduit, le NADPH, dont il est facile de suivre la vitesse de disparition en fonction du temps. Dans ces conditions, la cinétique d'oxydation du NADPH correspond à la cinétique d'oxydation du glutathion et, par voie de conséquence, à la cinétique de réduction de l'hydroperoxyde. Cependant, il n'existe pas de substrat hydroperoxyde spécifique de la pl.GPx ou de la c.GPx.

La discrimination entre l'activité de la c.GPx et l'activité de la pl.GPx est donc impossible à l'heure actuelle.

Compte-tenu du fait que l'activité spécifique de la c.GPx est approximativement dix fois supérieure à celle de la pl.GPx, une contamination de 1% en masse par la c.GPx introduira une surestimation de l'ordre de 10% de l'activité pl.GPx. Une légère hémolyse survenue dans un échantillon de sang peut ainsi introduire une erreur importante dans le calcul de l'activité pl.GPx du plasma circulant ou du sérum.

C'est donc un objectif de la présente invention de mettre au point une nouvelle méthode de dosage de la GPx permettant de distinguer la pl.GPx et la c.GPx et, en particulier, de doser spécifiquement la pl.GPx par une méthode immuno-enzymatique. Aucune méthode de dosage immunologique de la pl.GPx n'a encore été mise au point parce que l'importante homologie de séquence primaire de la pl.GPx et de la c.GPx provoque des réactions croisées entre les anticorps et les 2 types de molécules.

Pour contourner ce problème, la Demanderesse a
- évalué le pouvoir immunogène de divers oligopeptides synthétiques dérivés de la séquence publiée de la pl.GPx,
- sélectionné un oligopeptide et une série de variants de celui-ci, pour les utiliser sous forme de conjugués immunogènes pour induire, chez des animaux de laboratoire, la production d'anticorps spécifiques de la séquence choisie,
- démontré que lesdits anticorps reconnaissent spécifiquement la pl.GPx,
- mis au point une méthode d'immunodosage de la pl.GPx basée sur son immunocapture par lesdits anticorps,
- et incorporé lesdits anticorps dans un "kit" de dosage prêt à l'emploi.

La séquence publiée (Takahashi - voir liste de séquences p. 24, 25) de la pl.GPx a été étudiée à l'aide d'un logiciel permettant de définir le profil d'hydrophilicité et de flexibilité, et par conséquent de probabilité d'exposition à la surface de la molécule, de séquences peptidiques. Sur cette base, une série d'oligopeptides ont été choisis et synthétisés, d'une longueur de 13 à 16 acides aminés, présentant des séquences chevauchantes de manière à couvrir une grande partie de la séquence de la pl.GPx. La séquence entourant et comprenant le site actif a été éliminée à cause de sa trop grande homologie avec la séquence correspondante de la c.GPx

Ces oligopeptides ont été greffés comme haptènes sur de la serumalbumine bovine (BSA) pour être injectés à des lapins. Le sérum des lapins a été récupéré et le titre des anticorps a été évalué, dans un premier temps en système homologue, contre les peptides utilisés comme immunogènes puis contre la pl.GPx pour sélectionner les anticorps potentiellement utilisables dans un test de diagnostic. Les résultats ont été précisés par d'autres titrages et par des tests de compétition en présence d'antigène soluble.

La Demanderesse a ainsi sélectionné un oligopeptide, dont la séquence s'étend des résidus Glu 108 à Lys 120 de la séquence de la pl.GPx telle que publiée (même référence). Cet oligopeptide, SEQ ID N0: 1, constitue la base de la présente invention.

L'invention s'étend également à des variants de cette séquence dans lesquels, pour des raisons techniques qui peuvent faciliter la synthèse de l'oligopeptide ou produire une molécule plus stable, un ou deux acides aminés ont été remplacés par un acide aminé équivalent, c'est-à-dire sans que ce remplacement modifie la configuration spatiale ou le caractère hydrophile ou l'immunogénicité de l'ensemble de l'oligopeptide.

L'invention s'étend également à des variants de cette séquence, soit plus courts de quelques acides aminés, à l'extrémité N-terminale ou C-terminale ou les deux, soit plus longs de quelques acides aminés (de tels variants pouvant être obtenus par synthèse chimique ou par digestion enzymatique de la molécule naturelle), pour autant que lesdits variants permettent l'induction d'anticorps de même spécificité que la séquence choisie, c'est-à-dire des anticorps spécifiques de la pl.GPx.

Les oligopeptides ainsi définis sont utilisés comme immunogènes sous forme d'haptènes couplés sur une protéine porteuse, de type ovalbumine, sérumalbumine ...

La synthèse des oligopeptides choisis a été effectuée à l'aide d'un synthétiseur sur phase solide, additionnant les acides aminés de l'extrémité C-terminale vers l'extrémité N-terminale, dans des conditions classiques. En cours de synthèse, un acide aminé ne faisant pas partie de la séquence choisie a été ajouté en particulier la tyrosine, soit en C-terminal, soit en N-terminal, pour permettre le couplage de l'oligopeptide à la protéine porteuse.

Les conjugués immunogènes ainsi obtenus ont été injectés à des animaux de laboratoire (souris, rat, lapin, chèvre, etc...) selon un protocole classique pour obtenir des anticorps à haut titre.

La présente invention concerne également les anticorps dont la production résulte de l'utilisation des conjugués immunogènes définis plus haut. Ces anticorps sont purifiés par chromatographie d'affinité sur un gel de type Sepharose(r) greffé avec le même oligopeptide qui a servi d'haptène dans le conjugué immunogène, de manière à sélectionner les anticorps ayant la spécificité recherchée. Celle-ci est contrôlée par les mêmes titrages qui ont servi à choisir l'oligopeptide et qui montrent que les anticorps purifiés reconnaissent spécifiquement la pl.GPx naturelle, à l'exclusion des autres glutathion peroxydases.

La présente invention s'étend également à des anticorps monoclonaux présentant la même spécificité, induits selon des protocoles classiques en utilisant comme immunogène l'oligopeptide selon la présente invention et purifiés selon des méthodes classiques.

La présente invention concerne aussi une nouvelle méthode de dosage de la pl.GPx par immunocapture à l'aide des anticorps tels que définis plus haut, qui seront désignés ci-après "anticorps anti-peptide", suivie d'une révélation de la pl.GPx "capturée", par un second "anticorps révélateur", ce type de méthode étant couramment appelé "immunodosage en sandwich".

Cette méthode comprend donc :
- la fixation des anticorps anti-peptide à un support ;
- l'immunocapture par lesdits anticorps de la pl.GPx présente dans un échantillon de matériel biologique à doser ;
- la révélation de la pl.GPx immunocapturée, par un anticorps anti-pl.GPx naturelle marqué.

D'autres modes de mise en oeuvre du même principe de dosage par immunocapture peuvent aussi être envisagés et font partie de la présente invention.
- Selon un mode préféré de mise en oeuvre de la méthode, les anticorps anti-peptide sont fixés à un support qui peut être un puits de plaque ou de microplaque de type classique pour titrage en dilution, ou des billes de polystyrène ou tout autre matériel capable de fixer des anticorps.
- Les anticorps ainsi fixés permettent l'immunocapture spécifique de la pl.GPx présente dans l'échantillon à doser, celui-ci étant de préférence dilué en série.
- La pl.GPx ainsi capturée par l'anticorps anti-peptide est révélée à l'aide d'un second anticorps marqué pour augmenter la sensibilité de la détection. Le second type d'anticorps ou anticorps révélateur est obtenu après immunisation d'animaux avec de la pl.GPx purifiée ; ces anticorps sont purifiés par double extraction à l'acide caproïque puis au sulfate d'ammonium ; ils sont ensuite marqués par couplage avec une enzyme dont l'activité sera révélée en présence de son substrat, par colorimétrie (test de type ELISA) ou marqués par un isotope radioactif (test de type RIA) ou marqués par une substance fluorescente. Le second type d'anticorps peut éventuellement être un anticorps monoclonal.
- Le titre de la pl.GPx de l'échantillon à doser est déterminé par comparaison avec une courbe de référence établie avec un échantillon standard de pl.GPx purifiée.

L'avantage de la méthode selon la présente invention est sa très grande spécificité (c'est-à-dire la reconnaissance de la GPx plasmatique à l'exclusion des autres) qui est assurée par l'utilisation de l'anticorps anti-peptide sélectionné dans ce but.

La présente invention concerne également un "kit" ou trousse prête à l'emploi, pour la mise en oeuvre de la méthode de dosage décrite plus haut. Ce kit comprend :
- une plaque de titrage, sécable ou non, de préférence à 96 puits, dans lesquels l'anticorps anti-peptide a été fixé, selon une méthode classique, et recouvre toute la surface des puits,
- une solution de diluant des échantillons à doser, de préférence constituée d'une solution tamponnée (Tris ou phosphate), de NaCl, de protéine (caséine, ovalbumine ou sérumalbumine ...) à une concentration de 0,1 à 1 %, de détergent et d'agent conservateur (azidure de sodium ou merthiolate) et une solution de lavage de même composition mais sans protéines,
- un étalon de pl.GPx purifiée à partir de plasmas humains et lyophilisée,
- une solution d'anticorps révélateurs anti-pl.GPx marqués ; ces anticorps peuvent être polyclonaux ou monoclonaux et résultent d'une immunisation, selon un protocole classique, avec de la pl.GPx humaine purifiée ; ces anticorps sont marqués soit à la biotine, soit par une enzyme de révélation (comme la phosphatase alcaline ou la peroxydase de raifort). La solution d'anticorps est réalisée en tampon Tris ou phosphate et contient du NaCl 150 mM, de 0,1 à 1 % de protéine de surcharge (sérumalbumine, ovalbumine ou caséine), du glycérol et un agent conservateur,
- un substrat de révélation du marquage de l'anticorps, comme par exemple le pNPP (4-nitrophénylphosphate) pour la phosphatase alcaline, l'orthophénylènediamine pour la peroxydase ...

Le kit selon l'invention est utilisable pour tout dosage de la pl.GPx dans des fluides biologiques ou des extraits tissulaires, en particulier pour le diagnostic et le suivi thérapeutique de pathologies rénales ou hépatiques et d'états de carence nutritionnelle en sélénium.

Les applications potentielles incluent :
- Le diagnostic des états de carence en sélénium, en particulier chez les sujets malnutris, les personnes âgées et les patients soumis à une procédure de nutrition artificielle par voie entérale ou parentérale.
- Le diagnostic ou le suivi thérapeutique de l'insuffisance rénale, en particulier chez les patients dialysés.
- Le diagnostic précoce du rejet aigü de greffe hépatique ou rénale.
- Le diagnostic de certaines tumeurs hépatiques ou le suivi des traitements chimiothérapeutiques de ces tumeurs.

Les exemples suivants illustrent des aspects particuliers de la mise au point de l'invention ainsi que des modes de réalisation de celle-ci, sans toutefois en limiter la portée.

La liste de séquences, SEQ ID N0: 1 des p. 24-25, représente la séquence primaire de la glutathion peroxydase plasmatique déduite de la séquence nucléique de l'ADNc, telle que publiée par Takahashi K., Akasaka M., Yamamoto Y., Kobayashi C., Mizoguchi J. & Koyama J., (1990), J. Biochem., 108, 145-148. Les acides aminés soulignés sont communs à la pl.GPx et à la c.GPx). Dans la liste de séquences, le signe particulier Xaa pour l'aminoacide 73 représente la sélénocystéine.

Elle est jointe à la présente description pour faciliter la lecture mais elle n'est pas nécessaire à la réalisation de l'invention.

### Exemple 1 - Synthèse et sélection des oligopeptides.

La séquence en acides aminés de la pl.GPx déduite de l'ADNc et publiée par Takahashi et al. (J. Biochem. 108, 1990, 145-148 - voir SEQ ID N0: 1 -) a été analysée à l'aide d'un logiciel permettant de définir le profil d'hydrophilicité et de flexibilité de la séquence primaire.

Les oligopeptides suivants ont été choisis et synthétisés :

| N° de code | Longueur en acides aminés | Séquence* | % d'homologie avec la c.GPx |
|---|---|---|---|
| BXT-02-2003 | 15 | Lys 27 à Tyr 41 | 13 |
| BXT-02-2005 | 15 | Ser 37 à Glu 51 | 27 |
| BXT-02-2007 | 16 | Thr 47 à Gly 61 + Cys | 25 |
| BXT-02-2009 | 13 | Phe 56 à Val 67 + Cys | 31 |
| BXT-02-2011 | 15 | Tyr 79 à Gly 93 | 47 |
| BXT-02-2013 | 16 | Phe 99 à Ser 113 + Tyr | 69 |
| BXT-02-2015 | 14 | Glu 108 à Tyr 121 | 57 |
| BXT-02-2017 | 14 | Lys 120 à Gln 133 | 86 |
| BXT-02-2019 | 16 | Val 129 à Gly 143 + Tyr | 75 |
| BXT-02-2021 | 16 | Asp 139 à Lys 153 + Cys | 44 |
| BXT-02-2023 | 15 | Tyr 149 à Leu 163 | 33 |
| BXT-02-2025 | 15 | Thr 159 à Pro 173 | 20 |
| BXT-02-2027 | 16 | Tyr + Leu 169 à Phe 183 | 37 |
| BXT-02-2029 | 16 | Tyr + Ile 179 à Ile 193 | 81 |
| BXT-02-2031 | 15 | Gly 189 à Thr 203 | 47 |
| BXT-02-2033 | 16 | His 199 à Tyr 214 | 19 |
| BXT-02-2001 | 13 | Tyr 214 à Lys 226 | 8 |
| pl.GPx | 226 | | 44 |

| | | | |
|---|---|---|---|
| * La numérotation correspond à celle de la séquence publiée ; un acide aminé a parfois été ajouté (+) pour faciliter le couplage à la protéine porteuse. | | | |

Les oligopeptides ont été préparés dans les conditions suivantes :
a) Synthèses.
   Les conditions globales de synthèse sont extraites de protocoles décrits [Synthetic polypeptides as antigens, M.H.V. VAN REGENMORTEL, J.P. BRIAND, S. MULLER & S. PLAUE, 1988, Ed. R.H. Burdon et P.H. Van Knippenberg-Elsevier].
   Les séquences sont synthétisées sur phase solide dans le sens "extrémité C-terminale" vers "extrémité N-terminale".
   La pureté des acides aminés utilisés pour les synthèses est toujours supérieure ou égale à 99% (pureté CLHP).
   Les différents réactifs et solvants impliqués dans l'étape de coupure (acide fluorhydrique, éther, p-crésol et acide trifluoroacétique) sont caractérisés par une pureté d'au moins 99%.
   Après précipitation et filtration à l'éther, le peptide est solubilisé dans l'acide trifluoroacétique (TFA), le solvant évaporé et le peptide reprécipité à l'éther.
b) Purification.
   La purification des peptides est réalisée par CLMP^{*} à un débit compris selon le cas entre 10 et 200 ml/min. La colonne préparative utilisée contient une phase inverse (colonne C18). La séparation est assurée au moyen d'un gradient (25 à 60% de TFA) programmé sur 30 minutes.
   Les fractions issues de cette purification sont analysées par CLHP* sur colonne C18 et seules les fractions ayant la pureté requise sont lyophilisées.
   * CLMP = chromatographie liquide à moyenne pression
   CLHP = chromatographie liquide à haute pression.
c) Analyse par CLHP.
   L'analyse est réalisée sur colonne C18. Un gradient (10 à 60% de phosphate de triéthylamine -TEAP-) est programmé sur 15 minutes et la détection est assurée par un détecteur spectrophotométrique UV.
d) Préparation du conjugué.
   Les oligopeptides sont couplés sur une protéine porteuse pour être utilisés dans le cadre d'un programme d'immunisations d'animaux. Le conjugué est nécessairement constitué :
   * d'un haptène représenté par l'une des séquences oligopeptidiques précédemment décrites sur laquelle a généralement été greffé à l'extrémité soit N-, soit C-terminale, un acide aminé quelconque (comme par exemple la tyrosine). Le résidu supplémentaire est toujours incorporé dans la chaîne polypeptidique au cours de la synthèse peptidique.
      Cet acide aminé supplémentaire sert de lien entre la séquence proprement dite et un réactif bifonctionnel (tels la bisdiazobenzidine, le glutaraldéhyde, la m-maléimidobenzoyl-N-hydroxysuccinimide ester, le carbodiimide) utilisé pour coupler la structure polypeptidique à une protéine porteuse.
   * d'une protéine porteuse telle que l'ovalbumine ou OVA, l'albumine sérique bovine ou BSA, l'hémocyanine de patelle ou "keyhole limpet hemocyanin" (ou KLH).

- Couplage d'un oligopeptide et de la serumalbumine bovine par la bisdiazobenzidine (BDB) :
   L'utilisation de cet agent couplant est particulièrement recommandée dans le cas où le résidu situé à l'extrémité N- ou C-terminale est une tyrosine. Avant couplage à la BDB, le peptide peut avoir subi tout traitement destiné à protéger certains sites réactionnels comme par exemple une citraconylation réalisée dans le cadre de la protection de fonctions NH2 ou SH libres.
   Le couplage s'effectue selon le protocole suivant :
   Dissoudre le peptide dans du tampon HEPES 50 mM pH8,5 à raison de 0,2 mg/ml.
   Ajouter l'acide citraconique en plusieurs fois dans un excès molaire de 10 par rapport aux groupements réactionnels: le pH est ajusté à 8,5-9 avec de la soude 1M.
   Incuber une heure à température ambiante sous agitation modérée.
   Préparer la benzidine à une concentration de 5 mg/ml dans l'acide chlorhydrique 0,2M.
   Ajouter 3,5 mg de nitrite de sodium par ml de solution de benzidine, laisser incuber deux heures à 4°C sous agitation douce avant d'utiliser la BDB ainsi préparée.
   Toutes les étapes suivantes sont réalisées à 4°C.
   Procéder au couplage proprement dit en dissolvant 2,5 mg de serumalbumine dans 10 ml de tampon borate 0,16M pH9 contenant du NaCl 0,13M puis en ajoutant le peptide dans un rapport molaire BSA/peptide de l'ordre de 1/30 à 1/40. Laisser sous agitation pendant 10 minutes.
   Introduire dans le mélange ainsi constitué 100*µ*l de BDB et maintenir une agitation modérée pendant deux heures.
   Ajuster régulièrement le pH à 9 en additionnant de la soude 1M. Dialyser la solution afin d'éliminer l'excès de réactif et de peptide contre une solution de tampon PBS ou de solution physiologique. Au besoin, procéder préalablement à l'étape de déprotection des résidus traités à l'acide citraconique en dialysant la solution avec une solution d'acide acétique à 5%.

Les oligopeptides fixés sur de la serumalbumine bovine ont été utilisés pour immuniser des lapins, suivant un protocole classique :

On utilise une masse de conjugué correspondant à 100 µg de peptide par injection. L'injection est réalisée par voie intradermique et répétée 3 fois à 3 ou 4 semaines d'intervalle. Le sérum est collecté et la réponse en anticorps dirigés contre l'oligopeptide ("anticorps anti-peptide") est mesurée (par un test qui sera décrit dans l'exemple 4).

Pour choisir le peptide immunogène il faut mesurer à la fois le titre en anticorps anti-peptide et en anticorps anti-pl.GPx naturelle et vérifier que des anticorps anti-pl.GPx (induits chez le lapin) reconnaissent le peptide. Les résultats de ces tests apparaissent dans le tableau suivant.

| | Matériel utilisé pour sensibiliser les microplaques : | | |
|---|---|---|---|
| Sérum évalué : | peptides anti-peptide | pl.GPx anti-peptide | peptides anti-pl.GPx |
| N° de code des peptides | Titres | | |
| BXT-02-2003 | 1/2.000 | 1/1.000 | 1/100 |
| BXT-02-2005 | 0 | 0 | 0 |
| BXT-02-2007 | 1/3.000 | 1/1.000 | 0 |
| BXT-02-2009 | 0 | 0 | 0 |
| BXT-02-2011 | 1/10.000 | 1/1.000 | 1/300 |
| BXT-02-2013 | 1/.3.000 | <1/1.000 | 0 |
| BXT-02-2015 | 1/5.000 | 1/2.000 | 1/1.400 |
| BXT-02-2017 | 1/1.000 | 0 | 0 |
| BXT-02-2019 | 1/5.000 | 1/2.000 | 0 |
| BXT-02-2021 | 1/9.000 | 1/4.000 | 1/4.000 |
| BXT-02-2023 | 1/3.000 | 1/1.000 | 1/100 |
| BXT-02-2025 | 0 | 0 | 1/600 |
| BXT-02-2027 | 1/7.000 | 0 | 1/1.300 |
| BXT-02-2029 | 1/6.000 | 0 | 1/500 |
| BXT-02-2031 | 0 | 0 | 0 |
| BXT-02-2033 | 0 | 0 | 0 |
| BXT-02-2001 | 1/2.000 | 0 | 0 |
| PL.GPx naturelle | - | anti-pl.GPs 1/10.000 | - |

Après cette sélection, 4 séquences ont été retenues et soumises à d'autres analyses.
- Il a été vérifié que les anticorps correspondants n'avaient pas d'affinité pour la GPx érythrocytaire (c.GPx).
- Un dosage basé sur la compétition (voir exemple 5) entre reconnaissance de la pl.GPx insolubilisée sur plaque de titrage et reconnaissance de la pl.GPx en solution, par les anticorps anti-peptide a permis de vérifier si les anticorps sont capables de se lier à la protéine en solution.

Les concentrations de pl.GPx nécessaires pour atteindre environ 50 % d'inhibition sont les suivantes (entre parenthèses figurent les temps d'incubation pour la révélation colorimétrique à 37° et la densité optique ou absorbance lue en absence d'inhibiteur) :

| peptides | inhibition par pl.GPx en solution | |
|---|---|---|
| BXT-02-2003 | 1 *µ*g/ml | (50 min. 0,3 u. D.O.) |
| BXT-02-2015 | 5 *µ*g/ml | (30 min. 1,4 u. D.O.) |
| BXT-02-2019 | 5 µg/ml | (25 min. 0,6 u. D.O.) |
| BXT-02-2021 | pas d'inhibition | (40 min. 1,2 u. D.O.) |
| pl.GPx (référence) | 0,3 à 2 *µ*g/ml | (40 min. 1,3 u. D.O.) |

En considérant l'ensemble de ces résultats, le peptide BXT-02-2015 a été choisi ; sa séquence est indiquée ci-dessous pour mémoire : Elle correspond aux résidus 108 à 120 de la séquence publiée.

Cette séquence a été greffée sur l'ovalbumine par son extrémité C-terminale et par son extrémité N-terminale après addition d'un résidu tyrosine pour faciliter le couplage. On voit dans le tableau suivant que le second mode de greffage induit un titre beaucoup plus élevé.

| | Sensibilisation des microplaques | |
|---|---|---|
| | peptide | pl.GPx |
| Peptides | Titre du sérum anti-peptide : | |
| Glu 108 - Lys 120 - Tyr | 1/5.000 | 1/2.000 |
| Tyr - Glu 108 - Lys 120 | 1/15.000 | 1/12.000 |

### Exemple 2. Production d'anticorps polyclonaux anti-peptide.

Le peptide sélectionné, greffé sur de l'ovalbumine, comme décrit dans l'exemple précédent, a été injecté à des lapins selon le protocole suivant :
- injection : par voie intradermique, 1 ml par lapin d'un mélange vol/vol de 100 *µ*g de peptide en tampon PBS et d'adjuvant de Freund ;
- injections de rappel : dans les mêmes conditions (avec de l'adjuvant de Freund incomplet), à 3 à 4 semaines d'intervalle ;
- collecte de sang et centrifugation du sérum après une nuit à 4°C ou 2 heures à température ambiante ;
- purification des anticorps (voir exemple 3).

### Exemple 3. Purification des anticorps anti-pl.GPx par chromatographie d'affinité.

Pour récupérer sélectivement des anticorps spécifiques de la pl.GPx, les sera récupérés (exemple 2) ont été soumis à une purification par chromatographie d'affinité sur un gel greffé avec l'oligopeptide qui a servi d'immunogène, selon le protocole suivant :
* Greffage de 10 mg de peptide sur 1 ml de gel de Sépharose Haute Performance (PHARMACIA®) préactivé au moyen de la N-hydroxysuccinimide (colonne HiTrap NHS-activated). Désactivation des groupements actifs non couplés au ligand et lavage des ligands liés non spécifiquement par utilisation d'une solution saline d'éthanolamine 0,5M pH8,3 et d'une solution saline d'acétate 0,1M pH 4,0. Ces solutions contiennent toutes deux du NaCl 0,5M et sont utilisées en alternance sur des cycles de 6 ml de chaque solution à chaque étape (trois cycles). Equilibrer ensuite la colonne avec une solution de PBS avant de charger le sérum.
* Chargement d'environ 10 ml de sérum de lapin sur la colonne préalablement équilibrée au tampon PBS (phosphate-buffered saline).
* Lavage de la colonne avec le tampon PBS.
* Elution des anticorps spécifiques anti-peptide par passage de tampon citrate 0,1M pH2.
* Concentration des anticorps sur membrane d'ultrafiltration (seuil de coupure de 10 à 30.000).

Les anticorps ainsi purifiés reconnaissent spécifiquement la pl.GPx humaine et aucune réaction croisée significative n'est observée avec la c.GPx humaine. Ces anticorps sont capables de reconnaître la pl.GPx humaine fixée à un support insoluble ou en solution dans une phase liquide.

### Exemple 4. Titrage d'une solution d'anticorps.

La méthode de titrage peut s'appliquer aux sera collectés au cours de la sélection des peptides comme à l'anticorps spécifique purifié, selon l'exemple 3. La méthode repose sur la reconnaissance de l'antigène insolubilisé sur une microplaque de titrage et la révélation de l'anticorps fixé par un anti-anticorps marqué. On utilise de préférence des microplaques à 96 puits, classiquement utilisées pour les titrages dans divers domaines. Le titrage est effectué selon le protocole suivant :
* Sensibiliser les puits de la microplaque avec 100*µ*l de la solution d'antigène (oligopeptide ou pl.GPx naturelle purifiée) à 2 ou 1 *µ*g/ml respectivement (tampon de sensibilisation: Tris-HCl 10mM pH8,5, NaCl 100mM).
* Recouvrir la plaque d'une feuille autocollante puis l'incuber durant une nuit à 4°C ou 2 heures à 37°C
* Vider les puits et saturer les sites de fixation non spécifique avec une solution de protéine comme par exemple la gélatine 0,5% préparée avec du tampon Tris-HCl 50mM pH7,8, NaCl 150mM pendant 30 minutes à température ambiante.
* Vider puis laver les puits 3 fois avec la solution Tris-HCl 50mM à pH7,8 contenant du NaCl 150mM et du Tween 20 0,1%.
* Après élimination de la dernière solution de lavage, distribuer 100*µ* l de tampon de dilution (tampon Tris-HCl 50mM à pH7,8 contenant du NaCl 150mM, du Tween 20 0,1% et de la gélatine à 0,5%) dans l'ensemble des puits excepté le premier puits de chaque ligne. Chacun des puits non utilisé est réservé au dépôt de chacun des échantillons (sérum ou anticorps purifié) à tester et du sérum de contrôle (témoin négatif). Ces échantillons doivent souvent être dilués 50 à 200 fois avec le tampon de dilution. Le volume de dépôt est de 200*µ*l dans tous les cas. Les dilutions (dilution de 2 en 2) sont effectuées de puits en puits en transférant 100*µ*l de solution. Le transfert débute à partir du puits contenant les 200*µ*l d'échantillon de sérum à tester. Prévoir de réserver au minimum un puits pour le zéro interne du test, effectué au moment des lectures de densité optique: ce puits ne doit pas servir à diluer un sérum quel qu'il soit et ne doit donc contenir que le tampon de dilution.
   Les 100*µ*l prélevés à partir du dernier puits sont éliminés.
* Recouvrir la plaque d'une feuille autocollante puis mettre à incuber 2 heures à température ambiante.
* Vider la plaque et laver les puits par une série de 5 lavages successifs.
* Eliminer la dernière solution de lavage puis distribuer 100*µ*l de la solution de révélation, c'est-à-dire d'un conjugué anti-anticorps marqué (par exemple à la phosphatase alcaline). Cet anti-anticorps est spécifique de l'espèce d'où provient le sérum (lapin, souris, chèvre, etc...). La solution de conjugué est diluée avec le tampon de dilution (tampon Tris-HCl 50mM pH7,8 contenant du NaCl 150mM, du Tween 20 0,1% et de la gélatine à 0,5%).
* Recouvrir la plaque avec une feuille autocollante puis laisser incuber 1 heure à température ambiante.
* Vider la plaque et laver les puits par une série de 5 lavages successifs.
* Répartir 100*µ* l d'une solution de substrat, comme par exemple le paranitro-phénylphosphate dans le cas de l'utilisation de la phosphatase alcaline comme enzyme de révélation. Le pNPP est préparé à raison de 1 mg/ml de réactif dans un tampon Tris-HCl 0,1M pH9,5 contenant du NaCl 1,35M.
* Incuber la plaque à 37°C après l'avoir recouverte d'une feuille autocollante.
   La densité optique de chacun des puits est lue à 405 nm au bout de 20 minutes environ.
   Après enregistrement des signaux obtenus, les valeurs de densité optique sont représentées graphiquement en fonction du logarithme de la dilution du sérum.
   La valeur du titre est fixée comme étant égale à la valeur de dilution pour laquelle on obtient 50% du complexe antigène-anticorps.

### Exemple 5. Evaluation de la compétition entre la pl.GPx insolubilisée et la pl.GPx en solution pour la fixation des anticorps spécifiques.

Comme il a été mentionné dans l'exemple 1, l'anticorps anti-peptide a été choisi également pour sa capacité à fixer la pl.GPx en solution. Cette capacité est essentielle en vue de son utilisation dans un test de dosage par immunocapture, ce qui est le but de la présente invention. Pour évaluer cette capacité, un test de compétition entre la pl.GPx en solution et la pl.GPx adsorbée sur la microplaque de titrage est effectué selon le protocole suivant
a - La veille du dosage proprement dit :
   * Sensibiliser la plaque avec la pl.GPx à 1*µ*g/ml dans du tampon Tris-HCl 10mM pH8,5 contenant du NaCl 100mM.
   * Recouvrir la plaque à l'aide d'une feuille autocollante puis laisser incuber une nuit durant à 4°C.
   * Préparation des solutions contenant le mélange pl.GPx/anti-peptide.
   Remarques préliminaires :
   - La quantité d'anticorps à introduire est constante et n'est pas fixée au hasard. La dilution finale de la solution concentrée d'anticorps purifiés par affinité est déterminée à l'issue de son titrage (cf. protocole précédent): c'est la dilution pour laquelle on obtient arbitrairement une densité optique égale à 1 unité après incubation de 30 minutes à 37°C.
   - Le diluant des anticorps et de l'antigène libre (pl.GPx) est constitué de tampon Tris-HCl 50mM pH7,8 contenant du NaCl 150mM, du Tween 20 0,1% et de la caséine à 0,1%.
   - La concentration en inhibiteur peut varier entre 20 *µ*g/ml et 7,5 ng/ml. Le test décrit ci-dessous utilise des concentrations comprises entre 20 et 0,040 *µ*g/ml.
   Distribuer 100µl du diluant dans des tubes à essai numérotés de 2 à 10 et destinés à la préparation de la gamme
   du mélange anticorps - compétiteur (pl.GPx en solution). Introduire 200*µ*l de la solution de pl.GPx à 40 µg/ml dans le tube n°1. Transférer 100 µl de la solution contenue dans le tube n°1 dans le tube n°2, puis du tube n°2 vers le tube n°3 et ainsi de suite pour les tubes suivants. Les 100*µ*l prélevés du dernier tube sont éliminés.
   Préparer la solution d'anticorps à une concentration double de la concentration finale attendue dans le puits de la microplaque puis distribuer dans tous les tubes 100*µ*l de la solution d'anticorps et laisser incuber les mélanges durant une nuit à 4°C.
b - Le jour du test.
   * Vider les puits de la microplaque.
   * Saturer les sites de fixation non spécifique à l'aide d'une solution de protéine à 0,2%-1% (gélatine, ovalbumine, albumine sérique bovine).
   * Déposer 100µl de chaque point de gamme du tube n°1 au tube n°10 dans un puits distinct.
   * Laisser incuber 2 heures à température ambiante.
   * Vider les puits et réaliser 5 lavages successifs.
   * Eliminer la dernière solution de lavage puis distribuer 100µl de la solution de conjugué anti-anticorps marqué (par exemple à la phosphatase alcaline). Cet anti-anticorps est spécifique de l'espèce d'où provient le sérum (lapin, souris, chèvre, etc...). La solution de conjugué est diluée avec le tampon de dilution (tampon Tris-HCl 50mM pH7,8 contenant du NaCl 150mM, du Tween 20 0,1% et de la gélatine à 0,5%).
   * Recouvrir la plaque avec une feuille autocollante puis laisser incuber 1 heure à température ambiante.
   * Vider la plaque et laver les puits par une série de 5 lavages successifs.
   * Répartir 100µl d'une solution de substrat, comme par exemple le paranitro-phénylphosphate dans le cas de l'utilisation de la phosphatase alcaline comme enzyme de révélation. Le pNPP est préparé à raison de 1 mg/ml de réactif dans un tampon Tris-HCl 0,1M pH9,5 contenant du NaCl 1,35M. Incuber la plaque à 37°C après l'avoir recouverte d'une feuille autocollante.

La densité optique de chacun des puits est lue à 405 nm au bout de 30 minutes environ.
Soit B le signal obtenu en présence d'inhibiteur, B0 le signal obtenu en l'absence d'inhibiteur et bf le signal correspondant au bruit de fond (absence d'anticorps et de pl.GPx libre).
L'intensité du signal lu (B) est d'autant plus forte que l'inhibition est faible.
La représentation graphique de B-bf/Bo-bf en fonction du log. de la concentration en pl.GPx libre permet de visualiser l'influence de la concentration croissante de pl.GPx libre sur la reconnaissance de la pl.GPx fixée à la plaque par les anticorps anti-peptide spécifiques et permet de calculer la concentration de pl.GPx nécessaire pour donner une inhibition de 50 % de la fixation de l'anticorps sur la pl.GPx adsorbée.

### Exemple 6. Mise au point d'une méthode d'immunodosage spécifique de la pl.GPx.

Les anticorps anti-peptide purifiés comme décrit dans l'exemple 3 ont été utilisés pour réaliser un dosage immuno-enzymatique de type extraction-saturation ou "sandwich" dans lequel
- les anticorps anti-peptide sont adsorbés dans les puits de la microplaque (ce sont les anticorps "capteurs")
- la pl.GPx présente dans l'échantillon à doser est "immunocapturée" par les anticorps adsorbés
- la présence de la pl.GPx est révélée par un second anticorps "traceur" marqué avec une enzyme.

Le dosage a été effectué selon le protocole suivant :
- Les anticorps anti-pl.GPx utilisés comme anticorps traceurs proviennent de sera prélevés chez des lapins immunisés contre la protéine naturelle purifiée à partir de plasmas humains.
   Ces anticorps anti-pl.GPx sont purifiés par double extraction à l'acide caproïque puis au sulfate d'ammonium. Ils sont ensuite marqués à la biotine.
- Les anticorps anti-peptide (exemple 3) sont liés à un support insoluble qui peut être des puits de microplaque de titration, des billes de polystyrène ou tout autre matériel capable d'adsorber des anticorps. On utilise par exemple une microplaque à 96 puits. La plaque est sensibilisée avec 200 *µ*l d'une solution d'anticorps à 2-10 *µ*g/ml. Ces anticorps sont en solution saline (tampon Tris, phosphate, carbonate) à pH 7-9. L'incubation dure deux heures à 37°C ou une nuit à 4°C.
   Après sensibilisation, la plaque est lavée puis les sites de fixation non spécifique sont saturés au moyen soit d'une solution de sucres, soit d'une solution de protéine telle la caséine, la gélatine, la BSA ou l'ovalbumine.
   Cette étape dure environ de 30 minutes à deux heures à 37°C.
- La plaque est lavée puis l'échantillon à doser est déposé à raison de 100 *µ*l de solution par puits.
   La plaque est incubée une à deux heures à 37°C. Les puits sont alors vidés puis lavés avec le tampon de lavage.
- Un volume de 100*µ*l de solution d'anticorps anti-pl.GPx est déposé dans chaque puits. L'ensemble est mis à incuber pendant deux heures à 37°C.
- La plaque est à nouveau lavée puis 100 *µ*l de solution d'avidine marquée à la phosphatase alcaline sont déposés dans chaque puits. L'incubation à 37°C est alors maintenue une heure.
   Les puits sont vidés, lavés puis vidés avant d'introduire 100 *µ*l d'une solution de pNPP.
- Les densités optiques des solutions contenues dans chacun des puits sont lues à 405 nm avec un lecteur de plaques de microtitration de 96 puits.
   Un volume de 50*µ*l de solution d'arrêt (soude 1M) est ajouté lorsque la densité optique la plus élevée atteint 2 à 2,5 unités, soit après 20 à 30 minutes d'incubation à 37°C.

Le tableau suivant permet d'établir une courbe standard en portant la D.O. mesurée en fonction du logarithme de la concentration en pl.GPx.

| Concentration en pl.GPx | | Densité optique |
|---|---|---|
| ng/ml | log | |
| 600 | 2,78 | 2,47 |
| 300 | 2,48 | 1,97 |
| 150 | 2,18 | 1,32 |
| 75 | 1,88 | 0,77 |
| 37,5 | 1,57 | 0,43 |
| 18,8 | 1,27 | 0,22 |
| 9,4 | 0,97 | 0,10 |

Les échantillons biologiques à doser sont mesurés de la même manière et évalués par rapport à la courbe standard.

Si le lecteur spectrophotométrique de microplaque comprend un logiciel de calcul adapté, la représentation peut être effectuée directement par celui-ci.

### Exemple 7.

La méthode développée dans l'exemple précédent a servi de base à la mise au point d'un "kit" prêt à l'emploi pour doser la pl.GPx dans tout prélèvement sanguin ou tissulaire mis en solution.
Ce kit comporte l'ensemble des constituants suivants :
1- Plaque d'immunotitration à 96 puits sensibilisée avec l'anticorps anti-peptide purifié. Il peut s'agir d'une plaque sécable (barrettes de 8, 16, 24 ou 48 puits) ou d'une plaque non sécable. Dans tous les cas les barrettes ou la plaque sont conditionnées sous blister en présence d'un agent dessicant pour leur conservation.
2- Solution de diluant des échantillons: solution tamponnée (Tris, phosphate) contenant une protéine (telle que la caséine, la BSA, l'ovalbumine) à une concentration de l'ordre de 0,1% à 1%, un détergent ionique ou non ionique, et un agent conservateur (azidure de sodium ou merthiolate).
3- Etalon lyophilisé: il s'agit de la pl.GPx humaine purifiée à partir de plasmas humains et lyophilisée.
4- Tampon de lavage concentré: solution tamponnée (Tris, phosphate) contenant du NaCl, un détergent et un agent conservateur tels que ceux décrits prédédemment.
5- Solution d'anticorps traceurs marqués: il peut s'agir d'un anticorps monoclonal ou polyclonal anti-pl.GPx humaine obtenu par immunisation d'animaux avec de la pl.GPx humaine purifiée. Ces anticorps sont marqués à la biotine ou couplés à une enzyme de révélation (phosphatase alcaline ou PAL, peroxydase de Raifort ou HRP). Ces anticorps sont en solution dans un mélange contenant du tampon Tris ou phosphate, du NaCl (150mM), une protéine de surcharge (BSA, OVA, caséine) à 0,1-1 %, du glycérol et un agent conservateur.
6- Solution d'avidine couplée à une enzyme de révélation: cette solution n'est présente que lorsque l'anticorps traceur est biotinylé. Il peut s'agir d'avidine ou de streptavidine. Cette protéine peut être couplée à la PAL ou à l'HRP. Elle est en solution saline (Tris, phosphate) avec une protéine de surcharge (BSA, OVA, caséine) et un agent conservateur adapté au type de révélation (azidure de sodium proscrit pour la peroxydase).
7- Substrat(s) de révélation: pNPP dans le cas de la phosphatase alcaline et ortho-phénylènediamine (OPD) dans le cas de la peroxydase.
8- Tampon de dilution du substrat:
   * tampon basique (Tris , éthanolamine) pH9,5 contenant du chlorure de magnésium et un agent conservateur (azidure de sodium ou merthiolate) dans le cas de la phosphatase alcaline.
   * tampon acide (citrate pH5,5 par exemple) contenant le peroxyde d'hydrogène (environ 0,012%) et un agent conservateur (merthiolate) dans le cas de la peroxydase.
9- Solution d'arrêt:
   * solution de soude 1M contenant un chélateur de métaux comme l'éthylène diamine tétraacétate (EDTA) 0,1M dans le cas de la phosphatase alcaline;
   * solution d'acide sulfurique 1M dans le cas de la révélation à la peroxydase.

La mise en oeuvre du test est effectué selon le protocole suivant :
- Recueillir le sang veineux dans un tube en verre sans anticoagulant. Laisser reposer le sang au minimum deux heures avant de prélever le sérum. Centrifuger 10 minutes à 2000 x g et récupérer le sérum. Les échantillons de sérum sont stables 24 heures à 4°C. Au-delà, il est conseillé de congeler les échantillons à -20°C ou mieux à -80°C.
- Dilution : Les échantillons de sérum doivent être dilués dans la solution de diluant des échantillons. Une valeur de dilution comprise entre 1:40 et 1:80 est généralement suffisante.
- Suivre le protocole décrit dans l'exemple 6.

La sensibilité du test est excellente : à partir de 36 mesures simultanées du blanc (diluant d'échantillon) effectuées le même jour et sur la même plaque, la limite de détection déterminée est inférieure à 10 ng/ml.

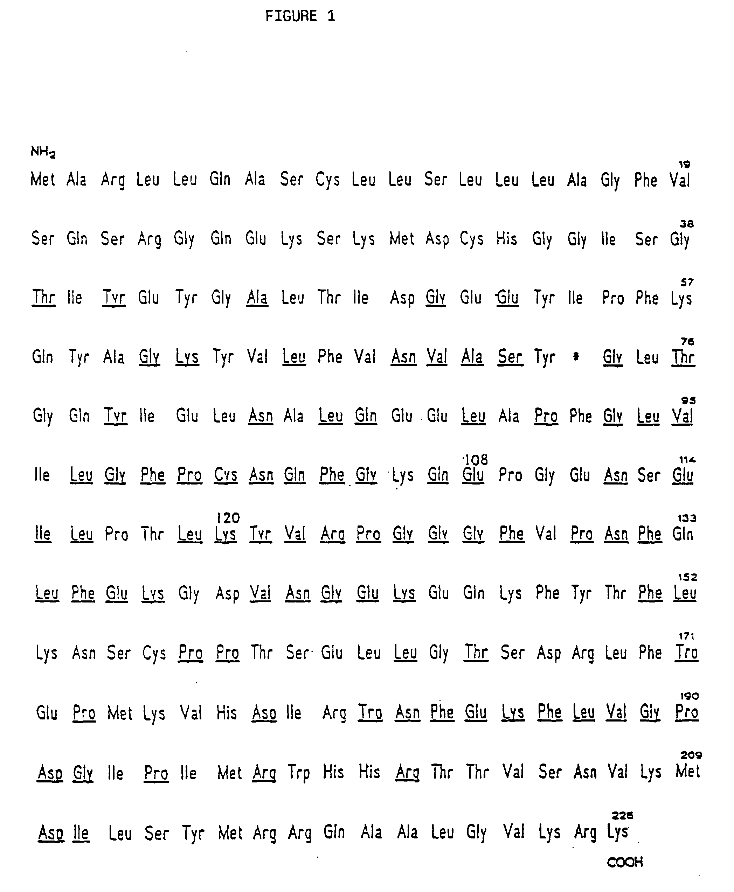

## Revendications

1. Oligopeptide de synthèse correspondant à la séquence comprise entre les résidus Glu 108 et Lys 120 de la séquence d'acides aminés de la glutathion peroxydase plasmatique (pl.GPx) humaine ayant la séquence de SEQ ID N0: 1.

2. Utilisation de l'oligopeptide selon la revendication 1 ou d'un variant de celui-ci pour lequel un ou deux acides aminés ont été remplacés par un acide aminé équivalent, ou un variant plus court de quelques acides aminés à l'extrémité N- terminale ou C- terminale ou les deux, ou un variant plus long de quelques acides aminés, pour autant que les dits variants plus courts ou plus longs que l'oligopeptide permettent l'induction d'anticorps spécifiques de la pl.GPx, pour la préparation de conjugués immunogènes destinés à la production d'anticorps par des animaux de laboratoire.

3. Utilisation selon la revendication 2, caractérisée en ce que le conjugué immunogène est constitué par un haptène représenté par la séquence oligopeptidique de la revendication 1, ou d'un variant de celle-ci, sur laquelle a été généralement greffé à l'extrémité soit N-, soit C-terminale, un acide aminé supplémentaire, en particulier la tyrosine, qui sert de lien entre la séquence proprement dite et un réactif bifonctionnel utilisé pour coupler la structure polypeptidique à une protéine porteuse.

4. Utilisation selon la revendication 3, caractérisée en ce que la protéine porteuse est choisie parmi le groupe consistant de l'ovalbumine, l'albumine sérique bovine, l'hémocyanine de patelle ou KLH.

5. Utilisation selon la revendication 3 ou 4, caractérisée en ce que l'oligopeptide ou son variant est couplé à de l'albumine sérique bovine.

6. Utilisation selon l'une des revendications 2 à 5 précédentes, caractérisée en ce que l'oligopeptide ou son variant comprend à l'extrémité N- ou C- terminale un amino acide supplémentaire constitué par la tyrosine.

7. Anticorps produits à l'aide des conjugués immunogènes préparés selon l'une des revendications 2 à 6, qui reconnaissent la pl.GPx naturelle.

8. Anticorps selon la revendication 7, caractérisés en ce - qu'ils sont purifiés par chromatographie d'affinité sur un gel greffé avec l'oligopeptide selon la revendication 1 et qu'ils reconnaissent spécifiquement la pl.GPx naturelle, à l'exclusion des autres glutathion peroxydases.

9. Méthode de dosage de la pl.GPx caractérisée en ce qu'elle comprend :
- la fixation des anticorps selon la revendication 7 ou 8 à un support,
- l'immunocapture par lesdits anticorps de la pl.GPx présente dans un échantillon de matériel biologique à doser,
- la révélation de la pl.GPx immunocapturée, par un anticorps anti-pl.GPx naturelle, marqué.

10. Trousse prête à l'emploi pour la mise en oeuvre de la méthode selon la revendication 9, caractérisée en ce qu'elle comprend :
- des plaques de titrage dont les puits sont recouverts d'anticorps selon la revendication 7 ou 8,
- des solutions de diluant et de tampon de lavage,
- un étalon de pl.GPx lyophilisée,
- une solution d'anticorps révélateurs anti-pl.GPx marqués,
- un substrat de révélation du marquage de l'anticorps.

## Patentansprüche

1. Synthese-Oligopeptid, das der Sequenz zwischen den Resten Glu 108 und Lys 120 der Aminosäuresequenz von menschlicher Plasma-Glutathion-peroxidase (pl.GPx) mit der Sequenz SEQ ID No:1 entspricht.

2. Verwendung des Oligopeptids nach Anspruch 1 oder einer Variante desselben, bei der eine oder zwei Aminosäure(n) durch eine äquivalente Aminosäure ersetzt sind, oder eine Variante, die am N-terminalen oder C-terminalen Ende oder an beiden Ende um einige Aminosäuren kürzer ist, oder eine Variante, die um einige Aminosäuren länger ist, damit diese Varianten, die kürzer oder länger als das Oligopeptid sind, die Induktion von pl.GPx-spezifischen Antikörpern ermöglichen, zur Herstellung von immunogenen Konjugaten, die für die Produktion von Antikörpern durch Labortiere bestimmt sind.

3. Verwendung nach Anspruch 2, dadurch gekennzeichnet, dass das immunogene Konjugat aus einem durch die Oligopeptidsequenz des Anspruchs 1 dargestellten Hapten oder einer Variante desselben besteht, an welcher ganz allgemein entweder am N- oder am C-terminalen Ende eine zusätzliche Aminosäure, insbesondere Tyrosin, aufgepfropft ist, die als Bindeglied zwischen der eigentlichen Sequenz und einem bifunktionellen Reagens dient, das zur Kopplung der Polypeptidstruktur an ein Trägerprotein verwendet ist.

4. Verwendung nach Anspruch 3, dadurch gekennzeichnet, dass das Trägerprotein ausgewählt ist aus der Gruppe bestehend aus Ovalbumin, Rinderserumalbumin, Patella-Hämocyanin oder KLH.

5. Verwendung nach Anspruch 3 oder 4, dadurch gekennzeichnet, dass das Oligopeptid oder seine Variante an Rinderserumalbumin gekoppelt ist.

6. Verwendung nach einem der vorhergehenden Ansprüche 2 bis 5, dadurch gekennzeichnet, dass das Oligopeptid oder seine Variante am N- oder C-terminalen Ende eine zusätzliche Aminosäure, bestehend aus Tyrosin, umfasst.

7. Antikörper, die mit Hilfe der nach einem der Ansprüche 2 bis 6 hergestellten immunogenen Konjugate erzeugt werden und natürliches pl.GPx erkennen.

8. Antikörper nach Anspruch 7, dadurch gekennzeichnet, dass sie mittels Affinitätschromatographie auf einem Gel gereinigt werden, auf welches das Oligopeptid nach Anspruch 1 gepfropft ist, und dass sie natürliche pl.GPx mit Ausnahme der anderen Glutathionperoxidasen spezifisch erkennen.

9. Verfahren zur Bestimmung von pl.GPx, dadurch gekennzeichnet, dass es die folgenden Schritte umfasst:
- die Fixierung der Antikörper nach Anspruch 7 oder 8 auf einem Träger,
- den Immuneinfang von in einer zu bestimmenden Probe aus biologischem Material vorhandener pl.GPx durch diese Antikörper,
- den Nachweis von immuneingefangener pl.GPx durch einen markierten natürlichen anti-pl.GPx-Antikörper.

10. Kit zur Verwendung bei der Durchführung des Verfahrens nach Anspruch 9, dadurch gekennzeichnet, dass er folgendes umfasst:
- Titrierplatten, deren Vertiefungen mit Antikörpern nach Anspruch 7 oder 8 bedeckt sind,
- Verdünnungs- und Waschpufferlösungen,
- einen lyophilisierten pl.GPx-Standard,
- eine Lösung von markierten anti-pl.GPx-Antikörpern zum Nachweis, und
- ein Substrat zum Nachweis der Markierung des Antikörpers.

## Claims

1. Synthetic oligopeptide corresponding to the sequence between residues Glu 108 and Lys 120 of the amino acid sequence of human plasma glutathione peroxidase (pl.GPx) whose sequence is SEQ ID N0: 1.

2. Use of the oligopeptide according to Claim 1 or a variant thereof for which one or two amino acids were replaced by an equivalent amino acid, or a variant which is shorter by a few amino acids at the N-terminal or C-terminal end, or both, or a variant which is longer by a few amino acids, so long as said variants that are shorter or longer than the oligopeptide allow for the induction of specific antibodies of the pl.GPx, for the preparation of immunogenic conjugates intended for the production of antibodies using laboratory animals.

3. Use according to claim 2, characterised in that the immunogenic conjugate is constituted by a hapten represented by the oligopeptide sequence of claim 1, or of a variant thereof, on which an additional amino acid, in particular tyrosine, has generally been grafted on the N-terminal or C-terminal end, acting as a link between the sequence itself and a bifunctional reactant used for coupling the polypeptide structure to a carrier protein.

4. Use according to claim 3, characterised in that the carrier protein is selected from the group consisting of ovalbumin, bovine serum albumin, hemocyanin of patelle or KLH.

5. Use according to claim 3 or 4, characterised in that the oligopeptide or any variant thereof is coupled to bovine serum. albumin.

6. Use according to one of the preceding claims 2 to 5, characterised in that the oligopeptide or any variant thereof comprises at the N-terminal or C-terminal end an additional amino acid constituted by tyrosine.

7. Antibodies produced by means of the immunogenic conjugates prepared according to one of claims 2 to 6, which recognise naturally occurring pl.GPx.

8. Antibodies according to claim 7, characterised in that - they are purified by affinity chromatography on a gel grafted with the oligopeptide according to claim 1
- and they specifically recognise naturally occurring pl.GPx, excluding other glutathione peroxidases.

9. A method for assaying pl.GPx, characterised in that it comprises:
- the binding of the antibodies according to claim 7 or 8 to a support,
- the immunocapture by the said antibodies of the pl.GPx present in a sample of biological material to be assayed,
- the revealing of the immunocaptured pl.GPx by a labelled antinatural pl.GPx antibody.

10. Ready-for-use set for the implementation of the method according to claim 9, characterised in that it comprises :
- titration plates whose wells are coated with antibodies according to claim 7 or 8,
- solutions of diluent and washing buffer,
- a freeze-dried pl.GPx standard,
- a solution of labelled anti-pl.GPx revealing antibodies,
- a substrate for revealing the antibody labelling.


### LISTE DE SEQUENCES

(1) INFORMATION GENERALE:
   (i) DEPOSANT:
      (A) NOM: BIOXYTECH
      (B) RUE: Z.A. des Petits Carreaux 2, avenue des Coquelicots
      (C) VILLE: BONNEUIL SUR MARNE
      (E) PAYS: FRANCE
      (F) CODE POSTAL: 94380
      (G) TELEPHONE: 49.80.45.64
      (H) TELECOPIE: 49.80.01.66
   (ii) TITRE DE L'INVENTION: Méthode d'immunodosage spécifique de la glutathion peroxydase plasmatique humaine, kit pour sa mise en oeuvre, oligopeptides et anticorps spécifiques de la méthode
   (iii) NOMBRE DE SEQUENCES: 1
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
   (vi) DONNEES DE LA DEMANDE ANTERIEURE:
      (A) NUMERO DE DEPOT: FR 93 10504
      (B) DATE DE DEPOT: 03-SEP-1993
(2) INFORMATION POUR LA SEQ ID NO: 1: -
   (i) CARACTERISTIQUES DE LA SEQUENCE:
      (A) LONGUEUR: 226 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 1:
   Met Ala Arg Leu Leu Gln Ala Ser Cys Leu Leu Ser Leu Leu Leu Ala
   1               5                   10                  15
   Gly Phe Val Ser Gln Ser Arg Gly Gln Glu Lys Ser Lys Met Asp Cys
               20                  25                  30
   His Gly Gly Ile Ser Gly Thr Ile Tyr Glu Tyr Gly Ala Leu Thr Ile
           35                  40                  45
   Asp Gly Glu Glu Tyr Ile Pro Phe Lys Gln Tyr Ala Gly Lys Tyr Val
       50                  55                  60
   Leu Phe Val Asn Val Ala Ser Tyr Xaa Gly Leu Thr Gly Gln Tyr Ile
   65                  70                  75                  80
   Glu Leu Asn Ala Leu Gln Glu Glu Leu Ala Pro Phe Gly Leu Val Ile
                   85                  90                  95
   Leu Gly Phe Pro Cys Asn Gln Phe Gly Lys Gln Glu Pro Gly Glu Asn
               100                 105                 110
   Ser Glu Ile Leu Pro Thr Leu Lys Tyr Val Arg Pro Gly Gly Gly Phe
           115                 120                 125
   Val Pro Asn Phe Gln Leu Phe Glu Lys Gly Asp Val Asn Gly Glu Lys
       130                 135                 140
   Glu Gln Lys Phe Tyr Thr Phe Leu Lys Asn Ser Cys Pro Pro Thr Ser
   145                 150                 155                 160
   Glu Leu Leu Gly Thr Ser Asp Arg Leu Phe Trp Glu Pro Met Lys Val
                   165                 170                 175
   His Asp Ile Arg Trp Asn Phe Glu Lys Phe Leu Val Gly Pro Asp Gly
               180                 185                 190
   Ile Pro Ile Met Arg Trp His His Arg Thr Thr Val Ser Asn Val Lys
           195                 200                 205
   Met Asp Ile Leu Ser Tyr Met Arg Arg Gln Ala Ala Leu Gly Val Lys
       210                 215                 220
   Arg Lys
   225